(19) Europäisches Patentamt
European Patent Office
Office européen des brevets

(11) EP 4 428 585 A1

(12) **EUROPEAN PATENT APPLICATION**
published in accordance with Art. 153(4) EPC

(43) Date of publication:
**11.09.2024 Bulletin 2024/37**

(21) Application number: **22890053.6**

(22) Date of filing: **07.11.2022**

(51) International Patent Classification (IPC):
***G02B 1/18*** *(2015.01)* ***G02C 7/02*** *(2006.01)*

(52) Cooperative Patent Classification (CPC):
**G02B 1/18; G02C 7/02**

(86) International application number:
**PCT/JP2022/041357**

(87) International publication number:
**WO 2023/080233 (11.05.2023 Gazette 2023/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **05.11.2021 JP 2021181428**

(71) Applicant: **Hoya Lens Thailand Ltd.**
**Pathumthani 12130 (TH)**

(72) Inventor: **KONO Shigetoshi**
**Tokyo 160-8347 (JP)**

(74) Representative: **Hoffmann Eitle**
**Patent- und Rechtsanwälte PartmbB**
**Arabellastraße 30**
**81925 München (DE)**

(54) **EYEGLASS LENS AND EYEGLASSES**

(57) Provided is a spectacle lens including a lens base material and an organic layer that contains a cationic organic compound.

EP 4 428 585 A1

## Description

[Technical Field]

The present invention relates to a spectacle lens and spectacles.

[Background Art]

PTL 1 discloses an antibacterial synthetic resin compact obtained by coating a synthetic resin surface with an antibacterial surface coating agent containing a zeolite which is ion-substituted with silver ions and a polymerizable compound having at least two (meth)acryloyloxy groups in a molecule as the main component, and curing the synthetic resin.

[Citation List]

[Patent Literature]

[PTL 1] Japanese Patent Application Publication No. H09-327622

[Summary of Invention]

[Technical Problem]

PTL 1 indicates that antibacterial activity is imparted by the surface coating agent to synthetic resin compacts that are used in building materials, various indoor structural materials, signboards, displays, lighting equipment, and the like.

In recent years, the need for antimicrobials has increased. Under such circumstances, if it is possible to impart a function capable of suppressing the proliferation of bacteria (that is, antibacterial activity) to spectacle lenses, it is possible to enhance the added value of the spectacle lenses.

An object of one aspect of the present invention is to provide a spectacle lens with antibacterial activity.

[Solution to Problem]

One aspect of the present invention relates to a spectacle lens including a lens base material and an organic layer that contains a cationic organic compound.

The spectacle lens can exhibit antibacterial activity when the cationic organic compound functions as an antibacterial agent.

[Advantageous Effects of Invention]

According to one aspect of the present invention, a spectacle lens with antibacterial activity can be provided.

[Description of Embodiments]

[Spectacle Lens]

The spectacle lens will be described in detail as follows.

In the present invention and the present description, an "organic layer" is a layer containing an organic substance and is preferably a layer containing an organic substance as a main component. A "main component" herein means a component that makes up the largest portion of the layer and, for example, may be a component that makes up the portion of not less than 50% by mass, not less than 60% by mass, not less than 70% by mass, not less than 80% by mass, or not less than 90% by mass of the layer in relation to the mass of the layer. For example, such a main component may be a component that makes up the portion of not more than 100% by mass, less than 100% by mass, not more than 99% by mass, not more than 98% by mass, not more than 97% by mass, not more than 96% by mass, or not more than 95% by mass of the layer in relation to the mass of the layer. The same applies to the main component of the inorganic layer, which will described later. As one embodiment of the organic layer, a hardened layer, that is, a so-called "hard coat layer", may be mentioned, and as another form of the organic layer, a foundation layer, which will be described later, may be mentioned. Details of these layers will be described later.

<Cationic Organic Compound>

The spectacle lens has an organic layer that contains a cationic organic compound. A "cationic organic compound" is an organic compound that can ionize to become a cation. The cationic organic compound can function as an antibacterial agent. Accordingly, the organic layer that contains a cationic organic compound serves as an antibacterial layer and can contribute to the spectacle lens exhibiting antibacterial activity. Furthermore, as a result of the study by the present inventor, the inventor has found that a spectacle lens including an organic layer that contains a cationic organic compound can exhibit excellent antibacterial activity and excellent light fastness. In the organic layer, part or whole of the cationic organic compound may be included in the form of salts or the form of a partially or fully ionized cation. The organic layer may contain a single type of cationic organic compound or two or more types of cationic organic compounds in any mixing ratio.

Examples of cationic organic compounds may include ammonium salts. An ammonium salt is a salt of an ammonium ion. An ammonium ion is a monovalent cation represented by "$NR_4^+$", wherein 4Rs each independently represent a hydrogen atom or a substituent. An ammonium ion salt in which three of four Rs are hydrogen atoms and the other one is a substituent is a primary ammonium salt. An ammonium ion salt in which two of four Rs are hydrogen atoms and the other two are substituents is a secondary ammonium salt. An ammonium ion salt in which three of four Rs are substituents and the other one is a hydrogen atom is a tertiary ammonium salt. An ammonium ion salt in which all four Rs are substituents is a quaternary ammonium salt. In an ammonium ion containing a plurality of Rs, a plurality of Rs are identical or different substituents. From the viewpoint that even better antibacterial activity can be exhibited, it is preferred that the cationic organic compound in the above organic layer is a quaternary ammonium salt.

Specific examples of quaternary ammonium salts may include an alkoxysilane-based quaternary ammonium salt. An alkoxysilane-based quaternary ammonium salt is a quaternary ammonium salt with an alkoxysilyl group. An alkoxysilyl group is a monovalent group represented by "$(R^{11}O)_3Si$-", wherein three $R^{11}$ each independently represent alkyl groups. For example, an alkoxysilyl group may be a methoxysilyl group "$(CH_3O)_3Si$-", an ethoxysilyl group "$(CH_3CH_2O)_3Si$-", and the like.

In one embodiment, the organic layer may contain a methoxysilane-based quaternary ammonium salt, that is, an ammonium salt with a methoxysilyl group. Specific examples of methoxysilane-based quaternary ammonium salts may include a methoxysilane-based quaternary ammonium salt represented by the following general formula (1).

[C1]

General formula (1)

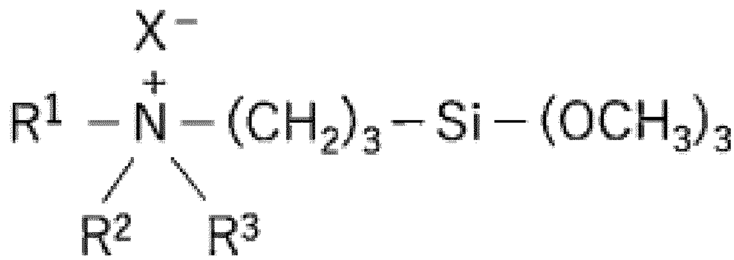

In the general formula (1), $R^1$ represents C12-24 alkyl group, $R^2$ and $R^3$ each independently represent C1-6 alkyl groups, and X represents a halogen ion or an organic carbonyloxy ion (an organic carboxylate ion).

Examples of the X in the general formula (1) may include halogen ions such as a chloride ion and a bromide ion and organic carbonyloxy ions (organic carboxylate ions) such as a methylcarbonyloxy ion (an acetate ion), an ethylcarbonyloxy ion (a propionate ion), and a phenylcarbonyloxy ion (a benzoate ion).

Examples of C12-24 alkyl groups represented by $R^1$ may include a dodecyl group, a tridecyl group, a tetradecyl group, a pentadecyl group, a hexadecyl group, a heptadecyl group, an octadecyl group, a nonadecyl group, an eicosyl group, an uneicosyl group, a doeicosyl group, a trieicosyl group, a tetraeicosyl group, and the like.

Examples of C1-6 alkyl groups represented by $R^2$ or $R^3$ may include a methyl group, an ethyl group, a propyl group, an isopropyl group, a butyl group, a pentyl group, a hexyl group, a cyclohexyl group, and the like.

Specific examples of methoxy silane-based quaternary ammonium salts represented by the general formula (1) may include octadecyldimethyl[3-(trimethoxysilyl)propyl]ammonium chloride, dodecyldimethyl[3-(trimethoxysilyl)propyl]ammonium chloride, dodecyldiisopropyl[3-(trimethoxysilyl)propyl]ammonium chloride, tetradecyldimethyl[3-(trimethoxysilyl)propyl]ammonium chloride, tetradecyldiethyl[3-(trimethoxysilyl)propyl]ammonium chloride, tetradecyldi-n-propyl[3-(trimethoxysilyl)propyl]ammonium chloride, pentadecyldimethyl[3-(trimethoxysilyl)propyl]ammonium chloride, pentadecyldiethyl[3-(trimethoxysilyl)propyl]ammonium chloride, pentadecyldi-n-propyl[3-(trimethoxysilyl)propyl]ammonium chloride, hexadecyldimethyl[3-(trimethoxysilyl)propyl]ammonium chloride, hexadecyldiethyl[3-(trimethoxysilyl)propyl]ammonium chloride, hexadecyldi-n-propyl[3-(trimethoxysilyl)propyl]ammonium chloride, octadecyldiethyl[3-(trimethoxysilyl)propyl]ammonium chloride, octadecyldi-n-propyl[3-(trimethoxysilyl)propyl]ammonium chloride, and the like. Among them, octadecyldimethyl[3-(trimethoxysilyl)propyl]ammonium chloride (the following formula (1)) is preferred from the viewpoint of bringing even better antibacterial activity by the spectacle lens.

[C2]

Formula (2)

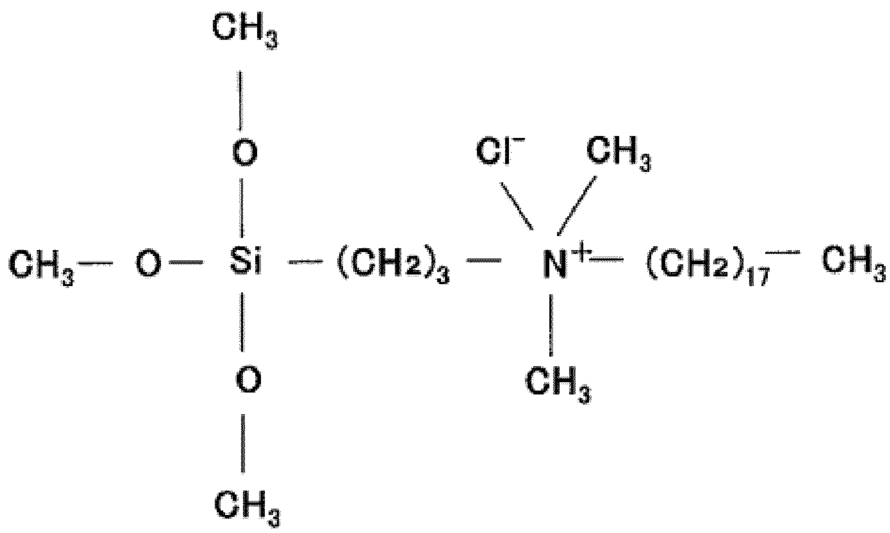

The cationic organic compound may be a commercially available product and may be a compound synthesized by a known method. Examples of commercially available quaternary ammonium salts may include Niccanon RB series, manufactured by Nicca Chemical Co., Ltd.

In the organic layer that contains a cationic organic compound, the content ratio of the cationic organic compound is preferably not less than 0.04% by mass and more preferably not less than 0.10% by mass in relation to the total mass of this organic layer as 100% by mass. A high content ratio of the cationic organic compound in the organic layer is preferable for the spectacle lens to exhibit even better antibacterial activity. For example, the content ratio of the cationic organic compound in the organic layer may be not more than 3.00% by mass. From the viewpoint of increasing the appearance quality of the spectacle lens (for example, prevention of fogging), the content is preferably not more than 2.50% by mass, and from the viewpoint of further increasing the light fastness and increasing the film strength, the content is more preferably not more than 2.20% by mass.

In one embodiment, the organic layer may contain one or more inorganic particles. Inorganic particles can contribute to increasing the film strength of the organic layer, increasing scratch resistance, and the like. Specific examples of inorganic particles may include various inorganic particles included in the curable composition described below, metal oxide particles included in the interference fringe-suppressing layer, and the like. When the organic layer that contains a cationic organic compound further contains inorganic particles, the content of the cationic organic compound in this organic layer may be not less than 0.5 parts by mass or not less than 0.7 parts by mass in relation to 100 parts by mass of inorganic particles. In this organic layer, the content of the cationic organic compound may be, for example, not more than 20.0 parts by mass in relation to 100 parts by mass of inorganic particles. In view of increasing the appearance quality of the spectacle lens (for example, prevention of fogging), the content is preferably not more than 18.0 parts by mass. Furthermore, from the viewpoint of further increasing light fastness and increasing film strength, the content is preferably not more than 15.0 parts by mass.

Examples of methods for forming the organic layer that contains a cationic organic compound may include a method including the steps of coating a composition that contains a cationic organic compound (hereinafter also referred to as a "composition for forming a cationic organic compound-containing organic layer") on a lens base material directly

or indirectly via another layer to form a coated layer and subjecting this coated layer to a curing process, a drying process, and the like. In the preparation of the composition for forming a cationic organic compound-containing organic layer, various components may be mixed simultaneously or sequentially in any order. In one embodiment, it is preferred to add a cationic organic compound to a mixture containing all components other than the cationic organic compound. The addition rate for adding the cationic organic compound to a composition containing one or more other components (for example, inorganic particles or the like) is preferably set to not faster than 0.5 g/min in view of suppressing the white turbidity of a composition for forming an organic layer. This is because the addition of the cationic organic compound at a relatively low speed is considered to be preferable for suppressing the agglomeration of various components by the addition of the cationic organic compound. It is preferred to suppress the white turbidity of the composition for forming an organic layer because suppressing the white turbidity leads to suppression of the occurrence of fogging in an organic layer formed from this composition and, as a result, can contribute to the improvement of appearance quality (suppression of the white turbidity) of a spectacle lens. For example, the addition rate mentioned above may be not slower than 0.1 g/min or may be below the values illustrated here.

For example, a layer containing the cationic organic compound can be one layer or two or more layers selected from the group consisting of cured layers, an interference fringe-suppressing layer, and a primer layer, which will be described below.

<Cured Layer>

The spectacle lens may include a cured layer formed by curing a curable composition. Such a cured layer is normally called a hard coat layer. In one embodiment, the organic layer that contains a cationic organic compound can be a cured layer formed by curing a curable composition. The organic layer (cured layer) that contains a cationic organic compound can be formed by curing a curable composition that contains a cationic organic compound.

The cured layer can be obtained by curing a curable composition containing, for example, silicon oxide particles (hereinafter, also referred to as "component (A)") and a silane compound (hereinafter, also referred to as "component (B)"). The curable composition may further contain a polyfunctional epoxy compound (hereinafter, also referred to as "component (C)").

From the viewpoint of achieving both abrasion resistance and optical properties, the particle size of the silicon oxide particles of the component (A) is preferably in the range of 5 nm to 30 nm.

The component (B) is a silane compound, preferably a silane compound having a hydrolyzable group, and more preferably a silane coupling agent having an organic group that bonds to a silicon atom and a hydrolyzable group.

Examples of hydrolyzable groups may include an alkoxy group, an aryloxy group, or a hydroxy group, and an alkoxy group is preferable.

A silane compound is preferably an organosilicon compound represented by the following general formula (I) or a hydrolyzate thereof.

$$(R^{21})_a(R^{23})_bSi(OR^{22})_{4-(a+b)} \cdots \quad (I)$$

In the general formula (I), a is 0 or 1, and b is 0 or 1, and preferably, a is 1, and b is 0 or 1.

$R^{21}$ represents an organic group having a functional group such as an epoxy group, including a glycidoxy group; a vinyl group; a methacryloxy group; an acryloxy group; a mercapto group; an amino group; and a phenyl group, and preferably represents an organic group having an epoxy group. The functional group may be directly bonded to a silicon atom or may be indirectly bonded via a linking group such as an alkylene group.

For example, $R^{22}$ is a hydrogen atom, an alkyl group, an acyl group, or an aryl group and preferably an alkyl group.

For example, the alkyl group represented by $R^{22}$ is a linear or branched C1-14 alkyl group. Specific examples thereof may include a methyl group, an ethyl group, a propyl group, a butyl group, and the like, and a methyl group or an ethyl group is preferable.

For example, the acyl group represented by $R^{22}$ is a C1-4 acryl group. Specific examples thereof may include an acetyl group, a propionyl group, an oleyl group, a benzoyl group, and the like.

For example, the aryl group represented by $R^{22}$ is a C6-10 aryl group. Specific examples thereof may include a phenyl group, a xylyl group, a tolyl group, and the like.

$R^{23}$ is an alkyl group or an aryl group.

For example, the alkyl group represented by $R^{23}$ is a linear or branched C1-6 alkyl group. Specific examples thereof may include a methyl group, an ethyl group, a propyl group, a butyl group, a pentyl group, a hexyl group, and the like.

For example, the aryl group represented by $R^{23}$ is a C6-10 aryl group. Specific examples thereof may include a phenyl group, a xylyl group, a tolyl group, and the like.

Specific examples of the component (B) may include the following silane compounds. Glycidoxymethyltrimethoxysilane, glycidoxymethyltriethoxysilane, α-glycidoxyethyltriethoxysilane, β-glycidoxyethyltrimethoxysilane, β-glycidoxyethyltriethoxysilane, α-glycidoxypropyltrimethoxysilane, α-glycidoxypropyltriethoxysilane, β-glycidoxypropyltrimethoxysilane, β-glycidoxypropyltriethoxysilane, γ-glycidoxypropyltrimethoxysilane, γ-glycidoxypropyltriethoxysilane, γ-glycidoxypropyltripropoxysilane, γ-glycidoxypropyltributoxysilane, γ-glycidoxypropyltriphenoxysilane, α-glycidoxybutyltrimethoxysilane, α-glycidoxybutyltriethoxysilane, β-glycidoxybutyltrimethoxysilane, β-glycidoxybutyltriethoxysilane, γ-glycidoxybutyltrimethoxysilane, γ-glycidoxybutyltriethoxysilane, δ-glycidoxybutyltrimethoxysilane, δ-glycidoxybutyltriethoxysilane, (3,4-epoxycyclohexyl)methyltrimethoxysilane, (3,4-epoxycyclohexyl)methyltriethoxysilane, β-(3,4-epoxycyclohexyl)ethyltrimethoxysilane, β-(3,4-epoxycyclohexyl)ethyltriethoxysilane, β-(3,4-epoxycyclohexyl)ethyltripropoxysilane, β-(3,4-epoxycyclohexyl)ethyltributoxysilane, β-(3,4-epoxycyclohexyl)ethyltriphenoxysilane, γ-(3,4-epoxycyclohexyl)propyltrimethoxysilane, γ-(3,4-epoxycyclohexyl)propyltriethoxysilane, δ-(3,4-epoxycyclohexyl)butyltrimethoxysilane, δ-(3,4-epoxycyclohexyl)butyltriethoxysilane, glycidoxymethylmethyldimethoxysilane, glycidoxymethylmethyldiethoxysilane, α-glycidoxyethylmethyldimethoxysilane, α-glycidoxyethylmethyldiethoxysilane, β-glycidoxyethylmethyldimethoxysilane, β-glycidoxyethylmethyldiethoxysilane, α-glycidoxypropylmethyldimethoxysilane, α-glycidoxypropylmethyldiethoxysilane, β-glycidoxypropylmethyldimethoxysilane, β-glycidoxypropylmethyldiethoxysilane, γ-glycidoxypropylmethyldimethoxysilane, γ-glycidoxypropylmethyldiethoxysilane, γ-glycidoxypropylmethyldipropoxysilane, γ-glycidoxypropylmethyldibutoxysilane, γ-glycidoxypropylmethyldiphenoxysilane, γ-glycidoxypropylethyldimethoxysilane, γ-glycidoxypropylethyldiethoxysilane, γ-glycidoxypropylvinyldimethoxysilane, γ-glycidoxypropylvinyldiethoxysilane, γ-glycidoxypropylphenyldimethoxysilane, γ-glycidoxypropylphenyldiethoxysilane, methyl silicate, ethyl silicate, n-propyl silicate, i-propyl silicate, n-butyl silicate, sec-butyl silicate, t-butyl silicate, tetraacetoxysilane, methyltrimethoxysilane, methyltriethoxysilane, methyltripropoxysilane, methyltriacetoxysilane, methyltributoxysilane, methyltripropoxysilane, methyltriamyloxysilane, methyltriphenoxysilane, methyltribenzyloxysilane, methyltriphenethyloxysilane, ethyltrimethoxysilane, ethyltriethoxysilane, vinyltrimethoxysilane, vinyltriacetoxysilane, vinyltrimethoxyethoxysilane, phenyltrimethoxysilane, phenyltriethoxysilane, phenyltriacetoxysilane, γ-chloropropyltrimethoxysilane, γ-chloropropyltriethoxysilane, γ-chloropropyltriacetoxysilane, 3,3,3-trifluoropropyltrimethoxysilane, γ-methacryloxypropyltrimethoxysilane, γ-mercaptopropyltrimethoxysilane, γ-mercaptopropyltriethoxysilane, β-cyanoethyltriethoxysilane, chloromethyltrimethoxysilane, chloromethyltriethoxysilane, N-(β-aminoethyl) γ-aminopropyltrimethoxysilane, N-(β-aminoethyl) γ-aminopropylmethyldimethoxysilane, γ-aminopropylmethyldimethoxysilane, N-(β-aminoethyl)-γ-aminopropyltriethoxysilane, N-(β-aminoethyl)-γ-aminopropylmethyldiethoxysilane, dimethyldimethoxysilane, phenylmethyldimethoxysilane, dimethyldiethoxysilane, phenylmethyldiethoxysilane, γ-chloropropylmethyldimethoxysilane, γ-chloropropylmethyldiethoxysilane, dimethyldiacetoxysilane, γ-methacryloxypropylmethyldimethoxysilane, γ-methacryloxypropylmethyldiethoxysilane, γ-mercaptopropylmethyldimethoxysilane, γ-mercaptopropylmethyldiethoxysilane, methylvinyldimethoxysilane, methylvinyldiethoxysilane, and the like.

As the silane compound, it is also possible to use a commercially available silane coupling agent. Specific examples of commercial products may include KBM-303, KBM-402, KBM-403, KBE402, KBE403, KBM-1403, KBM-502, KBM-503, KBE-502, KBE-503, KBM-5103, KBM-602, KBM-603, KBM-903, KBE-903, KBE-9103, KBM-573, KBM-575, KBM-9659, KBE-585, KBM-802, KBM-803, KBE-846, KBE-900, and the like, manufactured by Shin-Etsu Chemical Co., Ltd.

The component (C) is a polyfunctional epoxy compound. A polyfunctional epoxy compound is a compound having two or more epoxy groups in a molecule. The polyfunctional epoxy compound preferably has two or three epoxy groups in a molecule.

Specific examples of the component (C) may include the following polyfunctional epoxy compounds.

Aliphatic epoxy compounds such as 1,6-hexanediol diglycidyl ether, ethylene glycol diglycidyl ether, diethylene glycol diglycidyl ether, triethylene glycol diglycidyl ether, tetraethylene glycol diglycidyl ether, nonaethylene glycol diglycidyl ether, propylene glycol diglycidyl ether, dipropylene glycol diglycidyl ether, tripropylene glycol diglycidyl ether, tetrapropylene glycol diglycidyl ether, nonapropylene glycol diglycidyl ether, neopentyl glycol diglycidyl ether, diglycidyl ether of neopentyl glycol hydroxyhibaric acid ester, trimethylolpropane diglycidyl ether, trimethylolpropane triglycidyl ether, glycerol diglycidyl ether, glycerol triglycidyl ether, diglycerol diglycidyl ether, diglycerol triglycidyl ether, diglycerol tetraglycidyl ether, pentaerythritol diglycidyl ether, pentaerythritol triglycidyl ether, pentaerythritol tetraglycidyl ether, dipentaerythritol tetraglycidyl ether, sorbitol tetraglycidyl ether, diglycidyl ether of tris(2-hydroxyethyl) isocyanurate, and triglycidyl ether of tris(2-hydroxyethyl) isocyanurate, alicyclic epoxy compounds such as isophoronediol diglycidyl ether, bis-2,2-hydroxycyclohexylpropane diglycidyl ether and other, aromatic epoxy compounds such resorcin diglycidyl ether, bisphenol A diglycidyl ether, bisphenol F diglycidyl ether, bisphenol S diglycidyl ether, orthophthalic acid diglycidyl ester, phenol novolac polyglycidyl ether and cresol novolac polyglycidyl ether. As the component (C), a compound having two or three epoxy groups (difunctional or trifunctional epoxy compounds) is preferable from the viewpoint of an adhesive property to an adjacent layer or the lens base material.

Examples of commercial products of polyfunctional epoxy compounds may include "Denacol" series EX-201, EX-211, EX-212, EX-252, EX-313, EX-314, EX-321, EX-411, EX-421, EX-512, EX-521, EX-611, EX-612, EX-614, and EX-614B and the like, manufactured by Nagase ChemteX Corporation.

The above curable composition can be prepared by mixing, in addition to the components (A) to (C) described

above, optional components such as an organic solvent, a surfactant (leveling agent), and a curing catalyst according to need with the components described above.

The content ratio of the component (A) is preferably not less than 20% by mass, more preferably not less than 30% by mass, and still more preferably not less than 40% by mass, and preferably not more than 80% by mass, more preferably not more than 70% by mass, and still more preferably not more than 60% by mass when the total amount of solid contents in the curable composition (that is, the total of all components excluding the solvent) is set to 100% by mass.

The content of the component (B) is preferably not less than 5% by mass, more preferably not less than 10% by mass, and still more preferably not less than 15% by mass, and preferably not more than 80% by mass, more preferably not more than 70% by mass, and still more preferably not more than 60% by mass in relation to 100% by mass of the total amount of the solid contents in the curable composition.

The amount of the component (C) is, for example, not less than 0% by mass, preferably not less than 10% by mass, and still more preferably not less than 15% by mass, and preferably not more than 50% by mass, more preferably not more than 40% by mass, and still more preferably not more than 30% by mass in relation to 100% by mass of the total amount of the solid contents in the curable composition.

The filler/matrix ratio (hereinafter, also simply referred to as "F/M ratio") is preferably not less than 0.5, more preferably not less than 0.6, and still more preferably not less than 0.7 and preferably not more than 2.0, more preferably not more than 1.8, and still more preferably not more than 1.5. The F/M ratio means the mass ratio of the component (A) to the component (B) and the component (C)

[component (A)/(component (B) + component (C))].

As one example of a curable composition, a curable composition containing composite particles of stannic oxide and zirconium oxide may be mentioned. Regarding such a curable composition, the whole description of WO 2005/088352 can be referred to, and regarding the details of various components in the curable composition, the description in paragraphs [0007] to [0045] and the section of Examples of the same official gazette can be referred to.

By using a curable composition containing a cationic organic compound as the curable composition, an organic layer that contains a cationic organic compound can be formed as a cured layer formed by curing such a curable composition. It is preferred to set the addition rate for adding a cationic organic compound to be within the range stated above when such a curable composition is prepared for the reasons stated above. For example, the addition rate for adding a cationic organic compound to a curable composition containing inorganic particles can be set to be within the range stated above.

As a coating method of the curable composition, a known coating method such as a spin coating method, a dipping method, a spray coating method, or the like can be adopted. The same applies to the coating of compositions that are used to form various layers described below. Dip coating is preferred from the viewpoint of mass production of spectacle lenses. A curing process can be performed under light irradiation and/or heating treatment. Curing treatment conditions may be determined according to the types of various components contained in the curable composition or the composition of the curable composition. For example, the film thickness of a cured layer obtained by curing the curable composition is not smaller than 1 um and not larger than 100 um. The film thickness of the cured layer is preferably not smaller than 3 um and more preferably not smaller than 5 um from the viewpoint of enhancing the scratch resistance of the surface and is still more preferably not smaller than 8 um and further more preferably not smaller than 10 um from the viewpoint of significantly enhancing the scratch resistance, reducing the amplitudes of ripples, and significantly obtaining an interference fringe-suppressing effect and is preferably not larger than 80 μm, more preferably not larger than 60 μm, and still more preferably not larger than 50 μm.

<Foundation Layer>

The spectacle lens may have one or more foundation layers between the lens base material and another layer. For example, the foundation layer may be located between the lens base material and the cured layer or between the cured layer and an inorganic layer, which will be described layer. The spectacle lens may have one or two or more foundation layers. Specific examples of foundation layers may include a layer that functions as an interference fringe-suppressing layer (for example, an interference fringe-suppressing layer called a λ/4 layer) and a primer layer for increasing adhesive properties. The spectacle lens may include one or both of an interference fringe-suppressing layer and a primer layer between the lens base material and the inorganic layer or between the cured layer and the inorganic layer.

Interference Fringe-Suppressing Layer

The interference fringe-suppressing layer is a layer capable of suppressing the generation of interference fringes compared with a case where this layer is not included. A layer having an optical film thickness of 0.2 $\lambda$ to 0.3 $\lambda$ for light having a wavelength $\lambda$ of 450 nm to 650 nm is capable of functioning as the interference fringe-suppressing layer. The film thickness of the interference fringe-suppressing layer can be, for example, in the range of 50 nm to 100 nm in terms of physical film thickness.
For example, the interference fringe-suppressing layer can be formed by coating a dispersion containing at least metal oxide particles and a resin on the surface of the lens base material.
The metal oxide particles can play a role of regulating the refractive index of the interference fringe-suppressing layer. Examples of metal oxide particles may include particles of tungsten oxide (for example, $WO_3$), zinc oxide (ZnO), aluminum oxide (for example, $Al_2O_3$), titanium oxide (for example, $TiO_2$), zirconium oxide (for example, $ZrO_2$), tin oxide (for example, $SnO_2$), beryllium oxide (for example, BeO), antimony oxide (for example, $Sb_2O_5$), and the like. The metal oxide particles can be used singly or in combination of two or more thereof. Further, it is also possible to use complex oxide particles of two or more metal oxides. From the viewpoint of optical properties, the particle sizes of the metal oxide particles are preferably in the range of 5 nm to 30 nm. When the interference fringe-suppressing layer is the metal-containing layer and contains zinc oxide and/or zirconium oxide, these oxides are capable of playing a role of regulating the refractive index and a role of controlling the progress of the oxidation of silver.
Examples of resins for the interference fringe-suppressing layer may include at least one selected from a polyurethane resin, an acrylic resin, an epoxy resin, and the like. A polyurethane resin is preferable, and an aqueous resin composition containing a polyurethane resin, that is, an aqueous polyurethane resin composition is more preferable. For example, an aqueous polyurethane resin composition can be prepared by urethanizing a polyol compound and an organic polyisocyanate compound, and optionally together with a chain extender, in a solvent inert to the reaction and having a high affinity for water to prepare a prepolymer, neutralizing the prepolymer, and then dispersing the prepolymer in an aqueous solvent containing a chain extender to increase the molecular weight. Regarding such an aqueous polyurethane resin composition and a preparation method thereof, for example, paragraphs [0009] to [0013] of Japanese Patent No. 3588375, paragraphs [0012] to [0021] of Japanese Patent Application Publication No. H08-34897, paragraphs [0010] to [0033] of Japanese Patent Application Publication No. H11-92653, paragraphs [0010] to [0033] of Japanese Patent Application Publication No. H11-92655, and the like can be referred to. Furthermore, as the above aqueous polyurethane resin composition, a commercially available aqueous urethane can be used as it is or as a dilution diluted with an aqueous solvent according to need can be used. Examples of commercially available water-based polyurethane resin compositions may include "Evafanol" series (product name, commercially available from Nicca Chemical Co., Ltd.), "SuperFlex" series (product name, commercially available from DKS Co., Ltd.), "Adeka Bontighter" series (product name, commercially available from ADEKA Corporation), "Olester" series (product name, commercially available from Mitsui Chemicals Inc), "Bondic" series and "Hydran" series (product name, commercially available from DIC Corporation), "Impranil" series (product name, commercially available from Bayer AG), "Soflanate" series (product name, commercially available from Japan Soflan Co., Ltd.), "Poiz" series (product name, commercially available from Kao Corporation), "Sanprene" series (product name, commercially available from Sanyo Chemical Industries, Ltd.), "Aizerax" series (product name, commercially available from Hodogaya Chemical Co., Ltd.), "NeoRez" series (product name, commercially available from AstraZeneca), and the like.
The dispersion that is used to form the interference fringe-suppressing layer may contain an aqueous solvent. The aqueous solvent means a solvent containing water, is, for example, water or a solvent mixture of water and a polar solvent or the like, preferably water. The solid content concentration in the aqueous resin composition is preferably 1% by mass to 60% by mass and more preferably 5% by mass to 40% by mass from the viewpoint of liquid stability and film formability. The aqueous resin composition may also contain, aside from the resin component, additives such as an anti-aging agent, a dispersant, and a plasticizer according to need. Furthermore, commercially available aqueous resin compositions may be used after being diluted with a solvent such as water, alcohol, or propylene glycol monomethyl ether (PGM).
By using an aqueous resin composition that contains a cationic organic compound as the aqueous resin composition, an organic layer that contains a cationic organic compound can be formed as a foundation layer (interference fringe-suppressing layer) formed from such an aqueous resin composition. The foundation layer (interference fringe-suppressing layer) can be formed by coating an aqueous resin composition on a surface to be coated (for example, the surface of the lens base material) to form a coated layer and drying and removing at least a part of aqueous solvents by a drying process or the like to solidify the coated layer.

Primer Layer

For example, the primer layer can be an aqueous resin layer that is formed from an aqueous resin composition

containing a resin and an aqueous solvent. For example, the aqueous solvent that is contained in the aqueous resin composition is water or a solvent mixture of water and a polar solvent or the like, preferably water. The solid content concentration in the aqueous resin composition is preferably 1% by mass to 60% by mass and more preferably 5% by mass to 40% by mass from the viewpoint of liquid stability and film formability. The aqueous resin composition may contain, aside from the resin component, additives such as an anti-aging agent, a dispersant, and a plasticizer according to need. Furthermore, commercially available aqueous resin compositions may be used after being diluted with a solvent such as water, alcohol, or propylene glycol monomethyl ether (PGM).

The aqueous resin composition may include a resin component in a state of being dissolved or dispersed as particles (preferably colloidal particles) in an aqueous solvent. In particular, the aqueous resin composition is desirably a dispersion containing a resin component dispersed in a fine particle form in an aqueous solvent (preferably in water). In this case, the particle sizes of the resin component are preferably not larger than 0.3 um from the viewpoint of the dispersion stability of the composition. Furthermore, from the viewpoint of stability, the pH of the aqueous resin composition is preferably about 5.5 to 9.0 at 25°C. From the viewpoint of coating suitability, the viscosity at a liquid temperature of 25°C is preferably 5 mPa·S to 500 mPa·S, and more preferably 10 mPa·S to 50 mPa·S. For the resin, the earlier description about the interference fringe-suppressing layer can be referred to.

By using an aqueous resin composition that contains a cationic organic compound as the aqueous resin composition, an organic layer that contains a cationic organic compound can be formed as a foundation layer (primer layer) formed from such an aqueous resin composition. The foundation layer (primer layer) can be formed by coating an aqueous resin composition on a surface to be coated (for example, the surface of the interference fringe-suppressing layer or the surface of the lens base material) to form a coated layer and drying and removing at least a part of aqueous solvents by a drying process or the like to solidify the coated layer. For example, the film thickness of the primer layer can be in the range of 0.01 to 2.0 μm.

<Lens Base Material>

The lens base material of the spectacle lens can be a plastic lens base material or a glass lens base material. For example, the glass lens base material can be a lens base material made of inorganic glass. The lens base material is preferably a plastic lens base material because it is light weight, hard to break, and easy to handle. Examples of plastic lens base materials may include styrene resins such as (meth)acrylic resins, polycarbonate resins, allyl resins, allyl carbonate resins such as diethylene glycol bisallyl carbonate resins (CR-39), vinyl resins, polyester resins, polyether resins, urethane resins obtained by reacting an isocyanate compound and a hydroxy compound such as diethylene glycol, thiourethane resins obtained by reacting an isocyanate compound and a polythiol compound, and a cured product obtained by curing a curable composition containing a (thio)epoxy compound having one or more disulfide bonds in a molecule (generally referred to as a transparent resin). As the lens base material, an undyed lens (colorless lens) may be used, or a dyed lens (colored lens) may be used. For example, the refractive index of the lens base material may be about 1.60 to 1.75. However, the refractive index of the lens base material is not limited to the above range, and may be within the above range or may be above or below outside the above range. In the present invention and this specification, the refractive index shall mean a refractive index for light having a wavelength of 500 nm. In addition, the lens base material may be a lens having refractive power (so-called prescription lens) or a lens having no refractive power (so-called no-prescription lens).

The spectacle lens may include various lenses, such as a single focal lens, a multifocal lens, and a progressive power lens. The type of the lens is determined by the surface shape of both sides of the lens base material. In addition, the surface of the lens base material may be a convex surface, a concave surface, or a flat surface. In a general lens base material and spectacle lens, the object-side surface is a convex surface, and the eyeball-side surface is a concave surface. However, the present invention is not limited thereto.

<Inorganic Layer>

The spectacle lens has an inorganic layer on the lens base material. In the present invention and the present description, an "inorganic layer" is a layer containing an inorganic substance and is preferably a layer containing an inorganic substance as a main component. A "main component" herein means a component that makes up the largest portion of the layer. The main component is as described earlier about the organic layer. The inorganic layer can be a layer stacked on the surface of the lens base material at least via an organic layer that contains a cationic organic compound.

The inorganic layer can be a multilayer film of two or more inorganic layers in one embodiment. As such a multilayer film, a multilayer film including one or more high refractive index layers and one or more low refractive index layers can be exemplified. Such a multilayer film can be an antireflection film having the property of preventing the reflection of light having a specific wavelength or light in a specific wavelength range or a reflection film having the

property of reflecting light having a specific wavelength or light in a specific wavelength range. In the present invention and the present description, "high" and "low" of "high refractive index" and "low refractive index" are relative terms. That is, a high refractive index layer means a layer having a higher refractive index than a low refractive index layer included in the same multilayer film. In other words, a low refractive index layer means a layer having a lower refractive index than a high refractive index layer included in the same multilayer film. The refractive index of a high refractive index material configuring the high refractive index layer can be, for example, not smaller than 1.60 (for example, in the range of 1.60 to 2.40), and the refractive index of a low refractive index material configuring the low refractive index layer can be, for example, not larger than 1.59 (for example, in the range of 1.37 to 1.59). However, as mentioned above, the terms "high" and "low" in the phrases of high refractive index and low refractive index indicate relative magnitude; hence, the refractive index of the high refractive index material and that of the low refractive index material are not limited to the ranges mentioned above.

Specifically, examples of high-refractive-index materials for forming the high-refractive-index layer may include one oxide or a mixture of two or more oxides selected from the group consisting of zirconium oxide (for example, $ZrO_2$), tantalum oxide (for example, $Ta_2O_5$), titanium oxide (for example, $TiO_2$), aluminum oxide (for example, $Al_2O_3$), yttrium oxide (for example, $Y_2O_3$), hafnium oxide (for example, $HfO_2$), and niobium oxide (for example, $Nb_2O_5$). On the contrary, examples of low-refractive-index materials for forming a low-refractive-index layer may include one oxide or a mixture of two or more oxides or fluorides selected from the group consisting of silicon oxide (for example, $SiO_2$), magnesium fluoride (for example, $MgF_2$), and barium fluoride (for example, $BaF_2$). In these examples, the oxides and fluorides are indicated by stoichiometric composition, but the oxides and fluorides including oxygen or fluoride in an amount less than or more than the stoichiometric amount may also be used for the high refractive index material or the low refractive index material.

Preferably, the high-refractive-index layer is a film containing a high-refractive-index material as a main component, and the low-refractive-index layer is a film containing a low-refractive-index material as a main component. Such a film (for example, a deposition film) can be formed by film formation using a film-forming material (for example, a deposition material) containing the high-refractive-index material or the low-refractive-index material as a main component. Films and film-forming materials may contain impurities that are inevitably mixed and may contain other components, for example, other inorganic substances and known additive components that assist film formation to the extent that the functions of the main component are not impaired. Film formation can be performed by a known film-forming method, and in consideration of ease of film formation, the film formation is preferably performed by deposition and more preferably performed by vacuum deposition. For example, the antireflection film can be a multilayer film in which a total of 3 to 10 layers of high-refractive-index layers and low-refractive-index layers are alternately stacked. The film thickness of the high-refractive-index layer and the film thickness of the low-refractive-index layer can be determined according to the layer structure. Specifically, a combination of layers included in the multilayer film and the film thickness of each layer can be determined by an optical design simulation using a known technique based on the refractive index of the film-forming material for forming a high-refractive-index layer and a low-refractive-index layer, and the desired reflection property and transmission property to be brought about the spectacle lens by disposing the multilayer film. In addition, the multilayer film may include a layer containing, at any position, a layer containing a conductive oxide as a main component (conductive oxide layer) and preferably one or more layers of a deposition film of a conductive oxide formed by deposition using a deposition material containing a conductive oxide as a main component. The film thickness of each layer of the high refractive index layers and the low refractive index layers in the multilayer film can be, for example, 3 nm to 500 nm, and the total thickness of the multilayer film can be, for example, 100 nm to 900 nm. In the present invention and the present description, the film thickness is a physical film thickness unless particularly otherwise described.

The inorganic layer can bring an antireflection property with respect to light having a specific wavelength or light in a specific wavelength range to a spectacle lens when the inorganic layer functions as, for example, an antireflection film.

The spectacle lens may include one or more layers that can be normally included at any position. As one example of such a layer, a water-repellent layer may be mentioned. A water-repellent layer shall mean a layer that contributes to the exhibition of water repellency on the spectacle lens surface or a layer that contributes to the exhibition of better water repellency than a case without this layer. As one example of a water-repellent layer, a coated film containing an antibacterial agent (for example, silver particles) and a water-repellent agent described in WO 2021/060664 may be mentioned. Regarding such a water-repellent layer, the whole description of WO 2021/060664 can be referred to, and regarding the details of the antibacterial agent and the water-repellent agent, the paragraphs [0032] to [0061] of the same official gazette can be referred to, and the section of Examples of the same official gazette can also be referred to.

In the spectacle lens, the organic layer that contains a cationic organic compound can function as an antibacterial layer, and the spectacle lens can thereby exhibit antibacterial activity. In one embodiment, the organic layer that contains a cationic organic compound may also function as an antiviral layer.

[Spectacles]

One aspect of the present invention relates to spectacles having the spectacle lens described above. The details of the spectacle lens included in the spectacles are as described above. Regarding the spectacles, a known technique can be applied to the configuration of the frame and the like.

[Examples]

Hereinafter, the present invention will be described in more detail with reference to the examples. However, the present invention is not limited to embodiments shown in the examples.

[Comparative Example 1]

<Plastic Lens Base Material>

As a plastic lens base material, a plastic lens for spectacles, manufactured by HOYA Corporation (trade name: EYNOA, refractive index: 1.67) was used.

<Interference Fringe-Suppressing Layer (λ/4 Layer)>

To 305.0 g of methanol, 126 g of 4-hydroxy-4-methyl-2-pentanone (DAA) and 350.5 g of water were added, 217.5 g of a thermoplastic resin (SUPERFLEX 170, manufactured by DKS CO. Ltd.), 90.0 g of a sol-form material ($ZrO_2$ sol) containing HZ-407MH, manufactured by Nissan Chemical Corporation, dispersed in 40% by mass of methanol and 1.0 g of a leveling agent (Y-7006, manufactured by Dow Toray Co., Ltd.) were further added thereto, and the components were stirred for one hour at a liquid temperature of 20°C and treated with a filter, thereby obtaining a liquid for λ/4 layers. The obtained liquid for λ/4 layers was coated on the washed surfaces of a plastic lens base material by a dipping method and dried and solidified for 20 minutes in a dryer having an inner atmosphere temperature of 100°C, thereby forming λ/4 layers each with a film thickness (physical film thickness; the same applies hereinafter) of 85 nm on both surfaces of the lens base material.

<Primer Layer>

To 305.0 g of methanol, 126 g of 4-hydroxy-4-methyl-2-pentanone (DAA) and 350.5 g of water were added, 217.5 g of a thermoplastic resin (SUPERFLEX 170, manufactured by DKS CO. Ltd.) and 1.0 g of a leveling agent (Y-7006, manufactured by Dow Toray Co., Ltd.) were further added thereto, and the components were stirred for 24 hours at a liquid temperature of 20°C, thereby obtaining a primer liquid.
The obtained primer liquid was coated on the surfaces of the λ/4 layer by a dipping method and dried and solidified for 20 minutes in a dryer having an inner atmosphere temperature of 100°C, thereby forming primer layers each with a film thickness of 1 um on both surfaces of a lens base material.

<Hard Coat Layer>

A hard coat liquid was prepared by mixing 52 parts by mass of silica particles, 24 parts by mass of a silane coupling agent (KBM-403, manufactured by Shin-Etsu Chemical Co., Ltd., γ-glycidoxypropyltrimethoxysilane) and 24 parts by mass of a polyfunctional epoxy compound (DENACOL EX-321, manufactured by Nagase ChemteX Corporation, trimethylolpropane polyglycidyl ether).
The prepared hard coat liquid was coated on the surfaces of primer layers provided on both surfaces of a lens base material by a spray coating method, preliminarily cured by being heated in a heating furnace having an inner atmosphere temperature of 75°C for 20 minutes and, after raising the inner atmosphere temperature of the heating furnace to 110°C, mainly cured by being heated at the same temperature for two hours, thereby forming hard coat layers each with a film thickness of 5 um on both surfaces of the lens base material.

<Inorganic Layer (Multilayer Antireflection Film)>

Next, the lens base material on which the hard coat layers were formed was put in a vapor deposition device, and an eight-layered multilayer antireflection film, in which $SiO_2$ layers and $ZrO_2$ layers were alternately stacked, was formed on the surfaces of the hard coat layers by a vacuum vapor deposition method (total thickness: about 400 nm to 600 nm). The $SiO_2$ layer was a deposited film formed using silicon oxide as a deposition material, and the $ZrO_2$ layer

was a deposited film formed using zirconium oxide as a deposition material. Each deposition material was a deposition material composed only of the oxide described if inevitably incorporated impurities were excluded.

A spectacle lens having the λ/4 layer, the primer layer, the hard coat layer, and the multilayer antireflection film in this order on each of both surfaces of the lens base material was produced by the steps described above.

[Example 1]

A hard coat liquid was prepared in the manner described in Comparative Example 1.

While stirring the prepared hard coat liquid at a stirring speed of 500 rpm, a quaternary ammonium salt (Niccanon RB-40, manufactured by Nicca Chemical Co., Ltd.) was added to the hard coat liquid at an addition rate of 0.5 g/min, and the stirring was then continued for another 24 hours to prepare a quaternary ammonium salt. The amount of the quaternary ammonium salt added was set to an amount such that the content ratio of the quaternary ammonium salt should be 0.11% by mass and the content of the ammonium salt in relation to 100 parts by mass of silica particles should be 0.81 parts by mass in a hard coat layer formed.

A spectacle lens was prepared in the manner described in Comparative Example 1, except for the points stated above.

[Example 2]

A spectacle lens was prepared in the manner described in Example 1, except that the amount of the quaternary ammonium salt added to the hard coat liquid was set to an amount such that the content ratio of the quaternary ammonium salt should be 0.42% by mass and the content of the ammonium salt in relation to 100 parts by mass of silica particles should be 2.96 parts by mass in a hard coat layer formed.

[Antibacterial Tests]

Antibacterial tests on the spectacle lens of Example 1 and the spectacle lens of Example 2 were performed according to JIS Z 2801:2012. As a reference sample, a spectacle lens of Comparative Example 1 was used.

After a 50 mm × 50 mm test specimen (a test specimen cut out from each of the spectacle lenses) was placed in a sterilized petri dish, 0.4 mL of a bacterial solution containing $1.0 \times 10^5$ to $4.0 \times 10^5$ test bacteria (Staphylococcus aureus or Escherichia coli) was dropped onto a center of the test specimen, and the test specimen was covered with a polyethylene film cut into a 40 mm × 40 mm piece. After this petri dish was cultured at a relative humidity of not lower than 90% for 24 hours, a viable cell count per 1 $cm^2$ was measured, and the following antibacterial activity values were calculated.

Antibacterial activity value = Ut - At ≥ 2.0

Ut: Average value of the logarithmic number of the viable cell count per 1 $cm^2$ after a 24-hour culture of the unprocessed test specimen (reference sample)

At: Average value of the logarithmic number of the viable cell count per 1 $cm^2$ after a 24-hour culture of an antibacterial processed test specimen (example sample)

The Society of International sustaining growth for Antimicrobial Articles (SIAA) stipulates that, in a case where the antibacterial activity value of a product is not less than 2.0, the product has an antibacterial effect. The spectacle lens of Example 1 and the spectacle lens of Example 2 showed antibacterial activity values of not less than 2.0 to Staphylococcus aureus and Escherichia coli.

A spectacle lens was prepared in the same manner as Example 1, except that a plastic lens for spectacles, manufactured by HOYA Corporation (trade name: CR-39, refractive index: 1.50), was used as a lens base material, and no interference fringe-suppressing layer (λ/4 layer) was formed. The thus-prepared spectacle lens had an organic layer that contained a cationic organic compound as a hard coat layer, as with the spectacle lens of Example 1. Therefore, the thus-prepared spectacle lens is a spectacle lens that could function as an antibacterial lens as with the spectacle lens in Example 1.

[Example 3]

A spectacle lens was prepared in the manner described in Example 1, except that the amount of the quaternary ammonium salt added to the hard coat liquid was set to an amount such that the content ratio of the quaternary ammonium salt should be 1.66% by mass and the content of the ammonium salt in relation to 100 parts by mass of silica particles should be 11.83 parts by mass in a hard coat layer formed.

A water-repellent layer containing silver particles and having a film thickness of 15 nm was formed on inorganic layers

(multilayer antireflection films) on both surfaces of the formed spectacle lens in the manner described in paragraphs [0066] and [0067] of WO 2021/060554.

[Comparative Example 2]

A spectacle lens was prepared in the same manner as that described in Comparative Example 1, except that a water-repellent layer was formed on the inorganic layer in the manner described in Example 3.

[Antiviral Test]

An antiviral property test on the spectacle lens of Example 3 was performed in accordance with ISO 21702. As a reference sample, a spectacle lens of Comparative Example 2 was used.
A test virus liquid, 0.4 ml, is inoculated on the surface of a 50 mm ×50 mm test specimen (a test specimen cut out from each spectacle lens), and a cover film is put on the test specimen. Hereinafter, a test specimen on which the cover film is put is called a "sample".
The sample after inoculation of viral liquid is still stood (in contact) in an environment at a temperature of 25±1°C and a relative humidity of not less than 90% for 24 hours.
After the lapse of 24 hours, the virus is recovered from the surface of the sample, and a viral infectivity titer is measured by a plaque method. The following antiviral activity value is calculated.

$$\text{Antiviral activity value} = Ut\text{-}At \geq 2.0$$

Ut: Viral infectivity titer of an unprocessed test specimen (reference sample)
At: Viral infectivity titer of an antibacterial-treated test specimen (a sample of Examples)

The Society of International sustaining growth for Antimicrobial Articles (SIAA) stipulates that, in a case where the antiviral activity value of a product is not less than 2.0, the product has an antiviral effect. The spectacle lens of Example 3 showed an antiviral activity value of not less than 2.0 in an antiviral test in which an influenza virus (H3N2) was used as a virus strain.

[Example 4]

A spectacle lens was prepared in the manner described in Example 1, except that the amount of the quaternary ammonium salt added to the hard coat liquid was set to an amount such that the content ratio of the quaternary ammonium salt should be 2.26% by mass and the content of the ammonium salt in relation to 100 parts by mass of silica particles should be 16.14 parts by mass in a hard coat layer formed.

[Example 5]

A spectacle lens was prepared in the manner described in Example 1, except that the amount of the quaternary ammonium salt added to the hard coat liquid was set to an amount such that the content ratio of the quaternary ammonium salt should be 2.64% by mass and the content of the ammonium salt in relation to 100 parts by mass of silica particles should be 18.83 parts by mass in a hard coat layer formed.

[Example 6]

A hard coat liquid was prepared in the manner disclosed about Example 1 in paragraph [0091] of WO 2005/088352.
While stirring the prepared hard coat liquid at a stirring speed of 500 rpm, a quaternary ammonium salt (Niccanon RB-40, manufactured by Nicca Chemical Co., Ltd.) was added to the hard coat liquid at an addition rate of 0.5 g/min, and the stirring was then continued for another 24 hours to prepare a quaternary ammonium salt. The amount of the quaternary ammonium salt added was set to an amount such that the content ratio of the quaternary ammonium salt should be 2.26% by mass and the content of the ammonium salt in relation to 100 parts by mass of inorganic particles should be 3.30 parts by mass in a hard coat layer formed.
A spectacle lens was prepared in the manner described in Comparative Example 1, except for the points stated above.

[Reference Example 1]

A spectacle lens was prepared in the manner described in Example 1, except that silver particles with particle sizes of 2 to 5 nm were added instead of the quaternary ammonium salt. The amount of the silver particles added to hard coat liquid was set to an amount such that the content of the silver particle content ratio should be 1.89% by mass and the content of the ammonium salt in relation to 100 parts by mass of silica particles should be 3.79 parts by mass in a hard coat layer formed.

[Film Strength (Steel Wool Test)]

Steel wool tests were performed in the following manner on the surfaces of hard coat layers prepared in the manner described about Examples 1 to 6 and Reference Example 1.

A twenty-reciprocation abrasion resistance test was performed in a reciprocating friction and wear tester, manufactured by Shinto Scientific Co., Ltd., using steel wool (#00000, manufactured by BONSTAR) with a load of 2.0 kg. The surface of the hard coat layer after the test was visually observed, and the film strength was evaluated according to the following criteria.

(Evaluation Criteria)

A: No or almost no scratches are observed.
B: Few scratches are observed.
C: Obvious scratches are observed.
D: Scratches heavier than C are observed.

[Appearance Evaluation]

The appearance of each spectacle lens of Examples 1 to 6 and Reference Example 1 was observed by the eye, and the presence or absence of fogging was evaluated.

[Light Fastness (Yellowing Resistance)]

The light fastnesses of the spectacle lenses of Examples 1 to 4 and Reference Example 1 were evaluated by the following method.

The YI value (initial) of each spectacle lens was measured, and after the 0.32 kW irradiation for 300 hours in a Xenon Weather Meter XA25, manufactured by Suga Test Instruments Co., Ltd., the YI value (after irradiation) was measured. A ΔYI value was acquired as the difference of these,

$$[\text{(After irradiation)} - \text{(Initial)} = \text{(Variation)}].$$

The YI value was measured by the following method.

The direct incidence transmission spectroscopic characteristics at the optical center on the object-side surface (convex surface side) are measured from the object side of the spectacle lens. The YI value is obtained from the thus-obtained measurement results of the direct incidence transmission spectroscopic characteristics according to JIS K 7373:2006. Specifically, X, Y, and Z are calculated from the transmission spectrum obtained by measurement of the direct incidence transmission spectroscopic characteristics according to the formula (3) of JIS Z 8701:1999, and the YI value for the D65 light source is calculated from the calculation formula in Section 6.1 of JIS K 7373:2006. It can be said that, as the YI value becomes smaller, yellowing occurs less.

The light fastness (yellowing resistance) was evaluated based on the following evaluation criteria.

(Evaluation Criteria)

A: ΔYI value is not more than 0.5
B: ΔYI value is more than 0.5 and not more than 1

C: $\Delta YI$ value is more than 1 and not more than 2
D: $\Delta YI$ value is more than 2

The above results are shown in Table 1.

[Table 1]

| | Appearance evaluation | Light fastness (Yellowing resistance) | Film strength (Steel wool test) |
|---|---|---|---|
| Example 1 | No fogging | A | A |
| Example 2 | No fogging | A | A |
| Example 3 | No fogging | A | A |
| Example 4 | No fogging | B | B |
| Example 5 | Fogging was observed | - | D |
| Example 6 | No fogging | - | A |
| Reference Example 1 | Fogging was observed | D | A |

For the spectacle lens of Reference Example 1, antibacterial tests were performed according to the methods mentioned earlier. As a result, the antibacterial activity value of the spectacle lens of Referential Example 1 was not less than 2.0. However, as indicated in Table 1, fogging was observed in the appearance evaluation of the spectacle lens of Referential Example 1, and the evaluation result of light fastness was D.
The examples were compared. The spectacle lenses of examples 1 to 4 and 6, which contained a less amount of a quaternary ammonium salt in the hard coat layer than example 5, showed better results in appearance evaluation and film strength than example 5, in which the hard coat layer had a quaternary ammonium salt content ratio of 2.64% by mass and an ammonium salt content of 18.83 parts by mass in relation to 100 parts by mass of silica particles.
Finally, the above aspects are summarized.
According to one aspect, a spectacle lens including a lens base material and an organic layer that contains a cationic organic compound is provided.
The spectacle lens can exhibit antibacterial activity.
In one embodiment, the cationic organic compound may be a quaternary ammonium salt.
In one embodiment, the organic layer may further contain inorganic particles.
In one embodiment, the organic layer can contain not less than 0.5 parts by mass of the cationic organic compound in relation to 100 parts by mass of the inorganic particles.
In one embodiment, the organic layer can contain not less than 0.5 parts by mass and not more than 18.0 parts by mass of the cationic organic compound in relation to 100 parts by mass of the inorganic particles.
In one embodiment, the organic layer can have a content ratio of not less than 0.04% by mass of the cationic organic compound.
In one embodiment, the organic layer can have a content ratio of not less than 0.04% by mass and not more than 2.50% by mass of the cationic organic compound.
In one embodiment, the spectacle lens can have the organic layer on the lens base material and can further include an inorganic layer on the organic layer.
According to one form, there are provided spectacles having the spectacle lens.
The embodiments disclosed herein are only examples in all respects and should not be considered as restrictive. The scope of the present invention is not limited to the above description, but is defined by the scope of claims, and is intended to encompass equivalents to the scope of claims and all modifications within the scope of the claims.

[Industrial Applicability]

One aspect of the present invention is beneficial in the field of manufacturing spectacle lenses and spectacles.

**Claims**

**1.** A spectacle lens comprising a lens base material and an organic layer that contains a cationic organic compound.

2. The spectacle lens according to claim 1, wherein the cationic organic compound is a quaternary ammonium salt.

3. The spectacle lens according to claim 1 or 2, wherein the organic layer further contains inorganic particles.

4. The spectacle lens according to claim 3, wherein the organic layer contains not less than 0.5 parts by mass of the cationic organic compound in relation to 100 parts by mass of the inorganic particles.

5. The spectacle lens according to claim 3 or 4, wherein the organic layer contains not less than 0.5 parts by mass and not more than 18.0 parts by mass of the cationic organic compound in relation to 100 parts by mass of the inorganic particles.

6. The spectacle lens according to any one of claims 1 to 5, wherein the organic layer has a content ratio of not less than 0.04% by mass of the cationic organic compound.

7. The spectacle lens according to any one of claims 1 to 6, wherein the organic layer has a content ratio of not less than 0.04% by mass and not more than 2.50% by mass of the cationic organic compound.

8. The spectacle lens according to any one of claims 1 to 7, wherein the spectacle lens has the organic layer on the lens base material and further comprises an inorganic layer on the organic layer.

9. A pair of spectacles comprising the spectacle lens according to any one of claims 1 to 8.

## INTERNATIONAL SEARCH REPORT

International application No.
**PCT/JP2022/041357**

### A. CLASSIFICATION OF SUBJECT MATTER

***G02B 1/18***(2015.01)i; ***G02C 7/02***(2006.01)i
FI: G02B1/18; G02C7/02

According to International Patent Classification (IPC) or to both national classification and IPC

### B. FIELDS SEARCHED

Minimum documentation searched (classification system followed by classification symbols)

G02B1/18; G02C7/02

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2008-184556 A (ITO KOGAKU KOGYO KK) 14 August 2008 (2008-08-14) paragraphs [0008], [0071], [0079]-[0125], tables 1-3, fig. 1 | 1-9 |
| X | JP 5-179160 A (MITSUBISHI RAYON CO LTD) 20 July 1993 (1993-07-20) paragraphs [0032]-[0040], table 1 | 1-8 |
| Y | paragraphs [0032]-[0040], table 1 | 9 |
| Y | JP 2004-341052 A (ITO KOGAKU KOGYO KK) 02 December 2004 (2004-12-02) paragraph [0087] | 9 |
| Y | JP 9-227830 A (SEIKO EPSON CORP) 02 September 1997 (1997-09-02) paragraph [0066] | 9 |
| A | US 2021/0147648 A1 (3M INNOVATIVE PROPERTIES COMPANY) 20 May 2021 (2021-05-20) the whole document, fig. 1-3 | 1-9 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 December 2022** | **24 January 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/041357**

| Patent document cited in search report | Publication date (day/month/year) | Patent family member(s) | Publication date (day/month/year) |
|---|---|---|---|
| JP 2008-184556 A | 14 August 2008 | (Family: none) | |
| JP 5-179160 A | 20 July 1993 | (Family: none) | |
| JP 2004-341052 A | 02 December 2004 | (Family: none) | |
| JP 9-227830 A | 02 September 1997 | (Family: none) | |
| US 2021/0147648 A1 | 20 May 2021 | WO 2020/035802 A1<br>the whole document, fig. 1-3<br>EP 3837307 A1<br>CN 112585200 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H09327622 B
- WO 2005088352 A
- JP 3588375 B
- JP H0834897 B
- JP H1192653 B
- JP H1192655 B
- WO 2021060664 A
- WO 2021060554 A